Europäisches Patentamt

(19) European Patent Office

Office européen des brevets

(11) Publication number : **0 535 827 A1**

# (12) EUROPEAN PATENT APPLICATION

(21) Application number : **92308450.3**

(51) Int. Cl.⁵ : **A61K 37/02, A61K 47/44, A61K 9/66**

(22) Date of filing : **17.09.92**

(30) Priority : **28.09.91 GB 9120672**

(43) Date of publication of application :
**07.04.93 Bulletin 93/14**

(84) Designated Contracting States :
**AT BE CH DE DK ES FR GB GR IE IT LI LU NL PT SE**

(71) Applicant : **CIBA-GEIGY AG**
**Klybeckstrasse 141**
**CH-4002 Basel (CH)**

(72) Inventor : **Lowe, Phillip John**
**84 Congreve Road**
**Worthing, W. Sussex BN14 8EJ (GB)**
Inventor : **Hastewell, John Gilbert**
**44 York Close, Southwater**
**Horsham, W. Sussex RH13 7XJ (GB)**
Inventor : **Phillips, Judith Ann**
**46 Lynhurst Drive**
**Sevenoaks, Kent TN13 2HQ (GB)**

(74) Representative : **Sharman, Thomas et al**
**CIBA-GEIGY PLC. Patent Department, Central Research, Hulley Road**
**Macclesfield, Cheshire SK10 2NX (GB)**

(54) Calcitonin suspensions for oral administration.

⑰ The invention provides a pharmaceutical composition for oral administration to the gastrointestinal tract comprising solid calcitonin suspended in a non-aqueous liquid or solid and in the absence of an absorption enhancer.

EP 0 535 827 A1

The present invention relates to pharmaceutical compositions containing calcitonin.

Calcitonin is a 32 amino acid polypeptide hormone secreted by the parafollicular cells of the thyroid gland in mammals and by the ultimobranchial body of birds and fish. It is a potent drug for the treatment of Paget's disease, some aspects of hypercalcaemia, and for postmenopausal osteoporosis. Calcitonins of different origins, mainly salmon, pig, eel, and human, natural or sometimes modified, are currently used therapeutically.

The most convenient route for the delivery of drugs is via the gastrointestinal (GI) tract. For small drugs which are relatively hydrophobic and can be taken up effectively by the intestinal epithelium and transported to blood and/or lymph, this is often the route of choice. However, the permeability of the adult GI tract is such that large molecules, which may include bacterial toxins or antigenic proteins, are not absorbed to any great extent - although small but significant quantities of some hydrophilic macromolecules are taken up. In addition, the action of the intestinal and pancreatic proteases is such that proteinaceous or peptide drugs are digested to their constituent amino acids or oligopeptides and thus inactivated.

There are a growing number of proteins and peptides now being developed, often by biotechnological means, which need to be delivered to patients. These drugs are normally given by injection since they would be degraded in the intestinal tract after oral administration. However, since many of the disease indications for these drugs are not immediately life threatening or painful, the patient compliance for an injectable formulation can be very low. This is the case for calcitonin. There is therefore the need for a means whereby therapeutic proteins or peptides can be conveniently and safely delivered to patients via the oral route.

Many systems which are claimed to enhance the absorption of hydrophilic macromolecules from the intestine have been discovered. The enhancers include many different types of substances, including salicylates, surfactants such as bile acids and polyoxyethylene fatty acid esters or fatty acyl ethers, chelating agents such as ethylenediamine tetraacetic acid and solvents such as dimethylsulphoxide and ethanol.

Many of the known systems using enhancers for the intestinal absorption of peptide or protein drugs contain the active ingredient in the aqueous phase of the system, sometimes as an emulsion.

Formulations for rectal use often contain surfactants such as those listed above as enhancers in suppository bases.

We have now found that solid calcitonin can be suspended directly in a base without the need for enhancers and used for oral administration.

Accordingly the present invention provides a composition for oral administration to the gastrointestinal tract comprising solid calcitonin suspended in a non-aqueous liquid or solid and in the absence of an absorption enhancer.

The calcitonin may be salmon, pig, eel or human calcitonin and may be synthetic, extracted or produced by recombinant DNA technology.

The non-aqueous liquid or solid may be hydrophilic, hydrophobic or amphiphilic. Examples of suitable compounds include triglyceride vegetable or animal oils or fats, polyoxyethylene fatty acids or glycerides, glycolised and ethoxylated fatty acid esters, polyethylene glycols, propylene glycols or glycerol.

Preferred non-aqueous compounds are glycolised ethoxylated glycerides of natural oils such as apricot kernal oil, almond oil, peanut oil, olive oil and corn oil. The main fatty acids in these oils are oleic and/or linoleic. Especially preferred are the various unsaturated polyglycolised glycerides sold by Gattefosse, Saint-Priest, France under the Trade Marks Labrafil and Gelucire.

Preferably the non-aqueous compound is not a surfactant. If the non-aqueous compound is a solid, it should be one that softens or melts at body temperature and releases the calcitonin. Mixtures of non-aqueous compounds may be used.

The amount of calcitonin suspended in the non-aqueous solid or liquid may be from 0.01 to 10% weight for volume, preferably 0.1 to 10%.

The calcitonin is suspended or dissolved in the non-aqueous compound and the composition is blended and the particle size of the calcitonin reduced in known manner eg. using a homogeniser, emulsifier, grinder, sonicator, or a pestle and mortar. If the non-aqueous compound is solid at room temperature it is melted prior to suspension of the calcitonin. For oral administration the composition may then be filled into gelatine capsules.

If desired, one or more stabilizers may be added to the compositions to improve the storage stability. Useful materials include dextrin, acacia, carboxypolymethylene and colloidal aluminium hydroxide. When added they may be incorporated in amounts up to 10% (w/v), preferably from 0.5 to 6.5% of the total composition.

The compositions may also contain antioxidants and/or antimicrobial agents. Suitable antioxidants include propyl gallate, butylated hydroxyanisole, butylated hydroxytoluene, ascorbic acid or sodium ascorbate, DL- or D-$\alpha$-tocopherol and DL- or D-$\alpha$-tocopherol acetate. The antioxidant may be added in an amount up to 1% (w/v), preferably from 0.0001 to 0.3% of the total composition.

Antimicrobial agents may be used in amounts up to 3% (w/v), preferably from 0.5 to 2.5% of the total composition. Suitable antimicrobial agents include methylparaben, ethylparaben, propylparaben, butylparaben, phenol, dehydroacetic acid, phenylethyl alcohol, sodium benzoate, sorbic acid, thymol, thrimerosal, sodium dehydroacetate, benzyl alcohol, cresol, p-chloro-m-cresol, chlorobutanol, phenylmercuric acetate, phenylmercuric borate, phenylmercuric nitrate and benzylalkonium chloride.

Flavour enhancers, in appropriate amounts, may be added if desired.

The invention is illustrated by the following Examples.

Formulations are made in a homogeniser containing 0.34 mg/ml human calcitonin (CIBACALCIN) in various non-aqueous compounds as indicated in the Table below. The absolute bioavailability of the calcitonin is measured by the following procedure.

Wistar rats were anaesthetised prior to arterial cannulation. The abdomen was opened to allow access to the GI tract and a segment was isolated by ligation. Incisions were made and the distal end was tied off. The dose was administered through a needle at the proximal end and the ligature tightened. Great care was taken not to impair the blood low to the isolated segment. Aliquots of blood were withdrawn after administration and kept on ice at 0°C. These were prevented from clotting by the addition of heparin. The samples were centrifuged and the plasma decanted off.

Immediately after the 120 min blood sample had been collected, the experimental intestinal segment was fixed. The tissue was excised and placed in a cassette and immersed in the respective fixative. A control length of tissue was also fixed distal to the experimental segment. The tissues were embedded in paraffin wax, sectioned, stained with haematoxylin and eosin and examined for histological damage.

The bioactivity of the calcitonin was assayed by i.v. administration of 1.25 $\mu$g.kg$^{-1}$ calcitonin in 150 ml M NaCl. The blood was collected as above and assayed for plasma calcium.

Calcium concentrations were measured using a commercial colourimetric kit from SIGMA (procedure no 587). Calcitonin levels were determined using a commercial immunoassay (ELSA calcitonin, CIS Bio International).

The formulations used and the results obtained are shown in the Table:-

## Table

| Example | Non-aqueous Compound | Absolute Bioavailability |
|---------|----------------------|--------------------------|
| 1 | Oleic acid | 2.41% |
| 2 | Polyoxyethylene (20) sorbitan monooleate | 3.46% |
| 3 | Olive oil | 5.70% |
| 4 | Coconut oil | 1.27% |
| 5 | Soybean oil | 3.66% |
| 6 | Polyoxyethylene (23) castor oil | 1.77% |
| 7 | Gelucire 33/01 | 2.83% |
| 8 | Labrafil M1944CS | 2.51% |
| 9 | Labrafil M2125CS | 1.01% |
| 10 | Polyethylene glycol 300 | 9.49% |
| 11 | Polyethylene glycol 400 | 12.99% |
| 12 | Polyethylene glycol 600 | 0.74% |
| 13 | Propylene glycol | 7.22% |
| 14 | Glycerol | 1.62% |
| -- | Calcitonin alone | 0.26% |

Gelucire and Labrafil are Trade Names (Gattefosse, Saint-Priest, France) for derivatives of triglyceride oils obtained by hydrolysis and reaction with ethylene oxide.

As can be seen, the bioavailability of the intestinally administered calcitonin using a composition according to the invention is higher than the control in all cases.

**Claims**

1.  A pharmaceurical composition for oral administration to the gastrointestinal tract comprising solid calcitonin suspended in a non-aqueous liquid or solid and in the absence of an absorption enhancer.

2.  A composition as claimed in claim 1 in which the calcitonin is salmon, pig, eel, or human calcitonin.

3.  A composition as claimed in claim 1 or 2 in which the non-aqueous liquid or solid is hydrophobic, hydrophilic or amphiphilic.

4.  A composition as claimed in any proceeding claim in which the non-aqueous liquid or solid is a triglyceride vegetable or animal oil or fat, polyoxyethylene fatty acid or glyceride, glycolised acid ethoxylated fatty acid ester, polyethylene glycol, polypropylene glycol or glycerol.

5.  A composition as claimed in claim 4 in which the non-aqueous liquid or solid is a glycolised ethoxylated glyceride of a natural oil.

6.  A composition as claimed in claim 5 in which the natural oil contains oleic and/or linoleic acid.

7.  A composition as claimed in any preceding claim in which the amount of calcitonin is 0.01 to 10% weight for volume.

8.  A composition as claimed in any preceeding claim which is filled into gelatine capsules.

9.  A composition as claimed in any preceding claim which also contains a stabilizer, antioxidant, antimicrobial agent and/or flavour enhancer.

EP 0 535 827 A1

**European Patent Office**

# EUROPEAN SEARCH REPORT

Application Number

EP 92 30 8450

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X<br>Y | US-A-4 151 276 (FRANCIS B. CAULIN ET AL.)<br><br>* claims 1,6-8 *<br>* column 4, line 39 - line 66 *<br>--- | 1-4,7-9<br>5,6 | A61K37/02<br>A61K47/44<br>A61K9/66 |
| X | GB-A-1 354 526 (ARMOUR PHARMACEUTICAL COMPANY)<br>* claims 1,2,4 *<br>--- | 1-4 | |
| X | EP-A-0 438 147 (SCLAVO S.P.A.)<br>* claims 1,2,5,9 *<br>* page 3, line 40 - line 41 *<br>--- | 1-4 | |
| Y | FR-A-2 312 260 (CIBA-GEIGY AG.)<br>* claims 1,3,4 *<br>* page 4, line 35 - page 5, line 20 *<br><br>----- | 5,6 | |
| | | | **TECHNICAL FIELDS SEARCHED (Int. Cl.5 )**<br><br>A61K |

The present search report has been drawn up for all claims

| Place of search | Date of completion of the search | Examiner |
|---|---|---|
| THE HAGUE | 18 NOVEMBER 1992 | VENTURA AMAT A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

5